Europäisches Patentamt

European Patent Office

Office européen des brevets

(11) Publication number: 0 366 241
A2

## (12) EUROPEAN PATENT APPLICATION

(21) Application number: 89309026.6

(51) Int. Cl.5: G02B 21/34

(22) Date of filing: 06.09.89

(30) Priority: 04.10.88 US 253134

(43) Date of publication of application:
02.05.90 Bulletin 90/18

(84) Designated Contracting States:
AT BE CH DE ES FR GB GR IT LI NL SE

(71) Applicant: Fisher Scientific Company
711 Forbes Avenue
Pittsburgh, PA 15219(US)

(72) Inventor: Brigati, David John
3213 Adobe Drive
Edmond Oklahoma 73034(US)

(74) Representative: Williams, Trevor John et al
J.A. KEMP & CO. 14 South Square Gray's Inn
London WC1R 5EU(GB)

(54) Device with adsorbent surface and method.

(57) Devices such as microscope slides (110) bear a rigid polymer coating and an adsorbent polymer layer such as a 2 to 50 um thick clear nitrocellulose layer (113). Such nitrocellulose layers can be formed by applying to a positively charged glass surface a solution of nitrocellulose in methanol. Slide assemblies have two rigid slides whose facing surfaces are spaced by a gap 50 to 500 um thick. A first adsorbent polymer layer on one of the facing planar surfaces of the slide assembly can be used for immobilization of antigens, antibodies, nucleic acids, cells and other biological sample and reagent materials.

FIG.1

EP 0 366 241 A2

## DEVICE WITH ADSORBENT SURFACE AND METHOD

This application is related to our prior patent applications USSN 033,073, filed March 31, 1987, now U.S. Patent 4,777,020, and USSN 775,864, filed September 13, 1985, now U.S. Patent 4,731,335.

The present invention pertains to the field of devices with adsorbent surfaces for performing binding reactions, such as antibody-antigen binding or nucleic acid hybridization.

Microscope slides are typically rectangular glass objects having front and back faces or surfaces of a length (height) such as 75 mm or three inches (76.2 mm) and of a width such as 25 mm or one inch (25.4 mm). Such slides are made in a variety of thicknesses such as 0.9, 1.0 or 1.2 mm. It is common to frost part or all of the front face of the slide for a variety of purposes such as enabling the user to write sample identifying information on the slide. For such frosting or other reasons, a portion of the front face may be roughened or carved into. Other portions of the front face of the slide may remain sufficiently flat to be optically clear.

Partially coated slide described in U.S. Patents 4,481,246, 4,624,882 and 4,697,914 and typically sold under the SUPERFROST registered trademark has a polymeric coating of thickness under 20 microns (20 um) at an end on the surface of the slide (e.g., the end 10 mm).

U.S. Patent 4,731,335 (see also GB-A-2,180,647, published April 1, 1987) of Brigati describes modified microscope slides used in a method involving capillary action. Two microscope slides are arranged with front faces or surfaces opposite and held with such faces separated by a capillary gap (e.g., of about 50 - 500 um thickness). Such a gap is defined in some disclosed embodiments by a shim (tape or cover slip) fastened or held between the top portions of the apposite front faces. In another disclosed embodiment, the gap is defined by a coating on one of the slides. Further such embodiments are described in U.S. Patent 4,777,020 (see especially Figures 6A and 6B).

While such microscope slides have found use in immunological and nucleic acid hybridization analyses of tissue sections and other thin solid samples, the smooth glass surface provided on such slides is not conducive to adsorbing liquid samples. Instead, liquid samples (whether direct such as blood, serum or urine, or indirect such as extracts or materials run on columns or electrophoresis gels), are generally applied to adsorbent polymeric surfaces for such analyses. Exemplary of such surfaces are blotting filters made of nitrocellulose, polyester, polyamine (nylon) or of other natural, semi-synthetic or synthetic polymers. Such materials are illustrated in Schleicher & Schuell, "Products For Filtration, Separation and Life Science Research" pages F1 -F9 (Catalog 1985) (see references on page F3).

Processes employing such filter materials typically provide for multiple washing steps following each step whereat a liquid is applied to the adsorbent material. For example, in filter hybridizations, a probe reagent is applied to a filter containing an immobilized sample, incubated under denaturing and then renaturing conditions and then washed three times with buffers of three different stringencies to remove unhybridized probe. The same requirement for multiple washing applies after samples are spotted or blotted out of the filter material, after reagents of various kinds are applied to the adsorbed sample components and after reagents of various kinds (e.g., chromogens) are applied to immobilized labels (e.g., enzymes linked via antibodies or probes to the analyte component of the sample).

### Brief Description of the Invention

The present invention provides devices and methods for their preparation which result in adsorbent polymeric surfaces upon which samples can be immobilized and subsequently treated in multiple steps by capillary action. Such preparative methods include techniques which have more general applicability to the formation of very thin layers of adsorbent polymeric coatings on glass.

Thus the present invention provides, in a first form, a device comprising:

  a) a transparent sheetlike object having a planar front face, a linear lower edge and a thickness of about 0.5 to about 5.0 mm,

  b) a rigid polymer coating on a first portion of the planar front face of a thickness about 52 um to about 500 um, and

  c) an adsorbent polymer layer on a second portion of the planar front face of a thickness about 2 to about 50 um, the adsorbent polymeric layer having been deposited as a solution of a cellulosic or acrylamide polymer in a polar organic solvent and the thickness of the rigid polymer coating being at least 50 um greater than the thickness of the adsorbent polymer layer.

The present invention provides, in a second form, a slide assembly comprising:

  a) a first rigid slide having a planar front face, a linear lower edge and a thickness about 0.5 to about 5.0 mm,

b) a second rigid slide having a planar front face, a linear lower edge and a thickness about 0.5 to about 5.0 mm,

c) a first adsorbent polymer layer on a portion of the planar front face of the first rigid slide, the adsorbent polymeric layer having been deposited as a solution of a cellulosic or acrylamide polymer in a polar organic solvent and

d) spacer means for maintaining the first and second slides in a parallel and substantially vertical alignment, with the first adsorbent polymer layer being spaced from the second rigid slide by a gap of about 50 to about 500 um in thickness.

In a third form, the present invention provides a method for applying a very thin layer of an adsorbent polymeric coating on a glass surface which comprises the steps:

a) applying a solution of an adsorbent polymer selected from the group consisting of cellulosic polymers and acrylamide polymers in a polar organic solvent to a positively charged glass surface, and

b) drying the solution of step (a) on the positively charged glass surface, the concentration of the adsorbent polymer in the solution and the amount of solution applied being such that the dried layer formed by the drying step (b) is about 2 to about 50 um in thickness and forms a continuous coating.

Preferred forms of such method result in preferred forms of the device of the present invention because a rigid coating extending 50-500 micrometers further above the glass surface than the top of the adsorbent surface either is provided on the glass surface before the applying step (a) (or even before the glass surface is made to be positively charged) or is applied to the device after the applying step (a) (and preferably after the drying step (b)).

BRIEF DESCRIPTION OF THE DRAWINGS

Figure 1 is a front elevational view of a slide according to a first embodiment of the present invention;

Figure 2A is a end elevational view of a slide assembly consisting of two slides according to the first embodiment;

Figure 2B is a further enlarged end elevational view of the lower portion of the slide assembly of Figure 2A;

Figure 2C is a view similar to Figure 2B of the same lower portion of the slide assembly, but now taken in section through the middle of each slide 110 and 130;

Figure 3A is an end elevational view of liquid being drawn into the capillary gap formed by the slide assembly of Figure 2A;

Figure 3B is an end elevational view of liquid being drawn out of the capillary gap formed by the slide assembly of Figure 2A;

Figure 4A is a front elevational view of a partially-coated slide according to a second embodiment of the present invention.

Figure 4B is an end elevational view of the slide shown in Figure 4A and another slide inserted together into the holder of Figure 2A.

Figure 4C is a front elevational view similar to Figure 1 of a slide according to a third embodiment of the present invention;

Figure 5 is an end elevational view similar to Figure 4B, of two slides according to a third embodiment of the present invention inserted together into the same slide holder as is shown in Figures 2A and 4B.

DETAILED DESCRIPTION

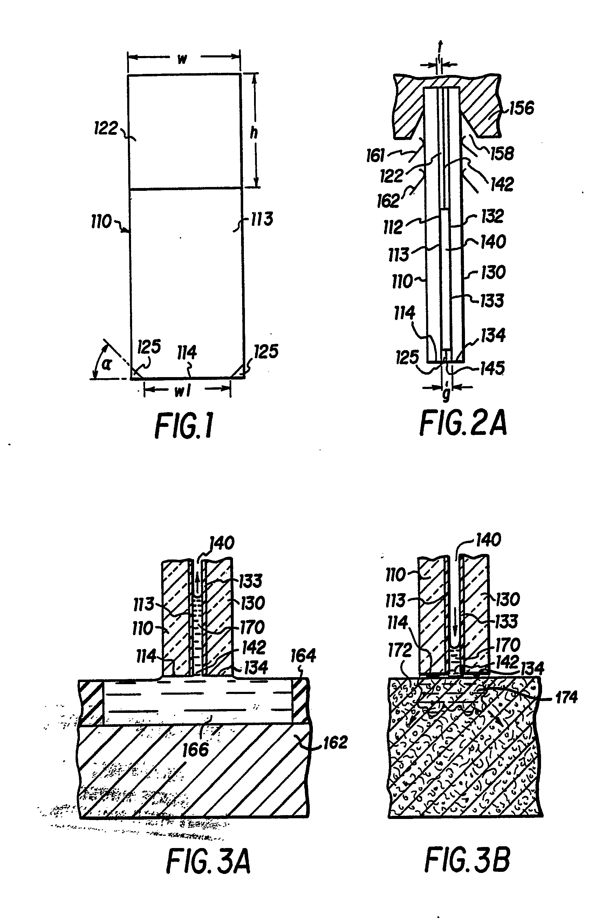

In Figure 1, a microscope slide 110 according to a first embodiment of the present invention is shown. The front face 112 of the slide 110 has an adsorbent polymeric coating 113 placed on the lower portion (44.4 mm out of a height of 74.2 mm) of the slide 110. A top coating 122 covers a portion of the front face of the slide (e.g., the top 31.8 mm out of a total height of 76.2 mm). Small triangular coating portions or raised islands 125 cover the bottom left and right corners of the front face of slide 110. Each of the coatings 122 and 125 are one-half the thickness of the desired gap of a slide assembly as illustrated in Figure 2A.

Representative dimensions for coating 122 are 31.8 mm high h, 25.4 mm wide w (the full width of slide 110) and 0.075 mm (75 um) thick t. Significant variations in height h and width w are permissable and, as described below, the thickness t can be 50 - 250 um . Representative dimensions for triangular coating portions 125 (raised islands) are 4 mm maximum height (along the outer left and right edges of slide 110), 4 mm maximum width (along bottom edge 114) and 75 um thickness. Substantial variations in the height and general shape of the raised islands 125 are permissable (as described more fully in application USSN 112,404 of Babbitt and Brigati filed October 26, 1987, copending); nevertheless it is preferred that each raised island extend from each corner inwardly along bottom edge 114 and upwardly along a side edge of slide 110.

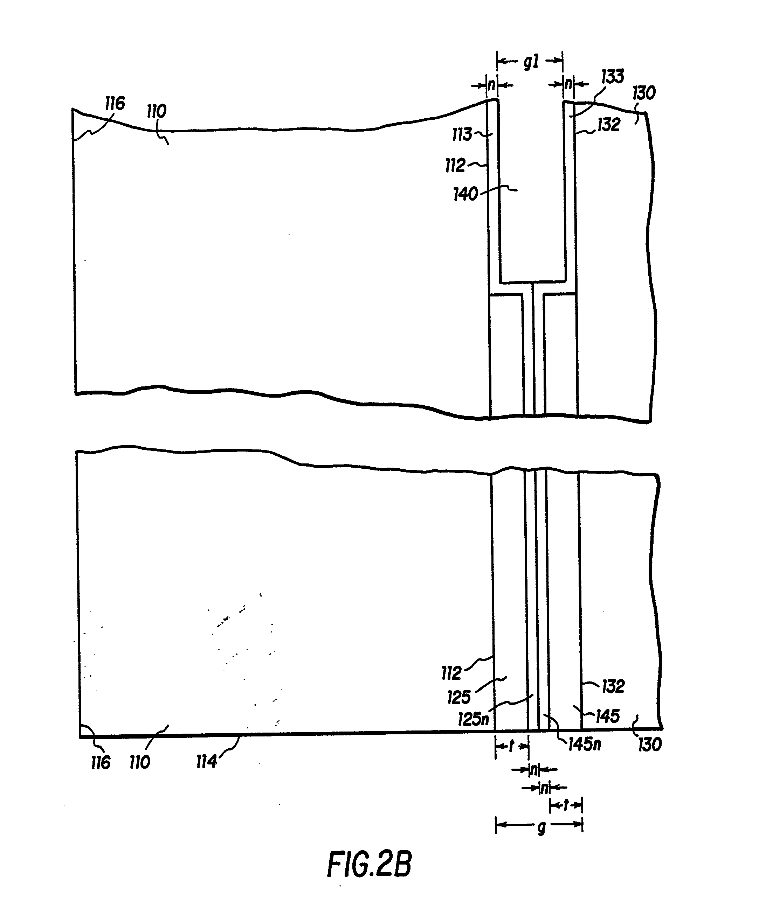

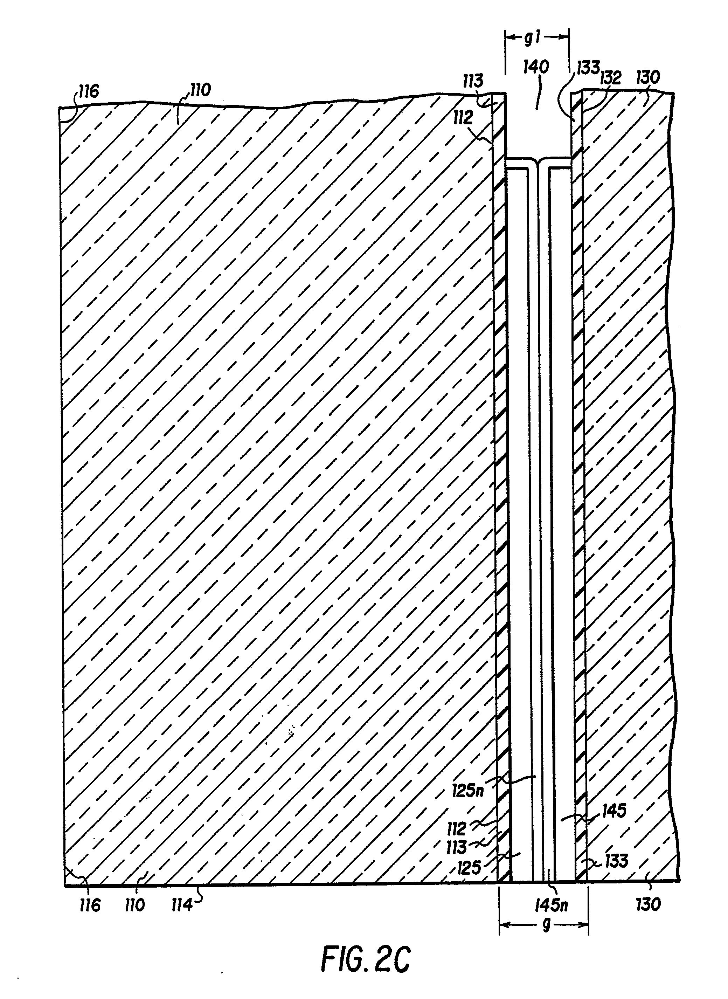

The adsorbent polymeric layer 113, in this first embodiment, has a thickness of 20 um. The importance of the relationship between this thickness and the thickness of the coatings 122 and 125 will become apparent in the description of Figures 2A, 2B and 2C, below. Exemplary methods for forming adsorbent polymeric layer of nitrocellulose are described and exemplified below.

Figure 2A is a side view of two identical slides 110 and 130 in a slide holder. Slides 110 and 130 together form a slide assembly when held together as shown in Figure 2A. Top coating 122 on slide 110 abuts against identical top coating 142 on slide 130. Bottom corner coating 125 on slide 110 abuts against bottom corner coating 145 in slide 130. Slides 110 and 130 are pressed together by clips 161 and 162 which are, respectively, above and below the midpoint of coatings 122 and 142. The tops of slides 110 and 130 are received within a slot 158 of alignment strip 156 of a slide holder described in U.S. patent application USSN 032,874 filed March 31, 1987, now U.S. Patent 4,801,431 issued January 31, 1989. That slide holder provides for each row of slide pairs two alignment strips with multiple slots (e.g., ten slots in each of two alignment strips for a row of ten slide pairs) positioned to engage the two top corners of each slide pair within a slot. Divider brackets of the slide holder extend downwardly on either side of each row of slide pairs. Clips on the divider brackets (illustrated as one top clip and one bottom clip on each divider bracket, or forty clips total for a row of ten slide pairs) hold the slides in a slide pair against each other and maintain the slide pairs in parallel alignment.

Referring again to Figure 2A, the horizontally-extending surface of the slot 158 maintains precise vertical alignment between slides 110 and 130. For the holder described in application 032,874, slots are provided near each of the two top corners of slides 110 and 130; and side walls are provided to maintain horizontal alignment between slides 110 and 130 in a direction into the page in the view of Figure 2A. In such slide holder, each alignment strip has multiple slots (illustrated as ten) so that multiple (ten) slide pairs can be held in a fixed array.

Figure 2B shows in greater magnification the end view of slides 110 and 130 in the vicinity of corner spots 125 and 145. The thickness of slide 110 from back face 116 to front face 112 is 1 mm (1000 um). The front face 132 of slide 130 is spaced from its back face by a similar 1 mm. Coating 125 of thickness 75 um is on the lowest 4 mm of front face 112; coating 145 of thickness 75 um is on the lowest 4 mm of front face 132. This thickness (75 um) is indicated in Figure 2B as t. A nitrocellulose layer 125n covers the front surface of corner spot 125; a nitrocellulose layer 145n covers the front surface of corner spot 145. The nitrocellulose layers 125n and 145n are each n units thick (illustrated as 20 um) and abut each other. Accordingly the spacing of front face 112 of slide 110 from front face 132 of slide 130 at the bottom (the height of lower edge 114) is g units, which is $2t + 2n$ or 190 um. Referring back to Figure 2A, the top coatings 122 and 142 are similarly coated with 20 um thick nitrocellulose layers so that the spacing between face 112 and face 132 is a uniform 190 um.

Referring back to Figure 2B, it can be seen that, at the near end of the slides 110 and 130, the gap between faces 112 and 132 is filled with corner spots 125 and 145 and layers 125n and 145n. Above corner spots 125 and 145 (that is 4 mm above lower edge 114) a gap 140 is present. Nitrocellulose layers 113 and 133, each of thickness n, cover glass surfaces 112 and 132, respectively adjacent to gap 140. If this n is also 20 um, then the gap 140 is of thickness $g1$, which is $g - 2n$ = 150 um.

Referring now to Figure 2C the interior of the lower end of the slide assembly can be seen. The numerals 110, 112, 113, 114, 116, 125, 125n, 130, 132, 133, 140, 145 and 145n all refer to the same elements as in Figures 2A and 2B. Now glass slides 110 and 130 are shown in section, as are nitrocellulose layers 113 and 133. The corner spots 125 and 145 and nitrocellulose layers 125n and 145n visible in this view are in the far corner of the lower edge of the slide assembly.

In the view of Figure 2C, the gap 140 surrounded by nitrocellulose layers 113 and 133 extends down to the bottom of the slide assembly (the height of lower edge 114). Referring to Figure 1, it can be seen that gap 140 would extend to the bottom over the width w1, and flares out to the full width w at a 45 degree angle alpha over the bottom 4 mm of the slide assembly. In this first embodiment, the gap 140 is surrounded by layers 113 and 133 throughout, and is a uniform thickness of 150 um up to the base of coating 122.

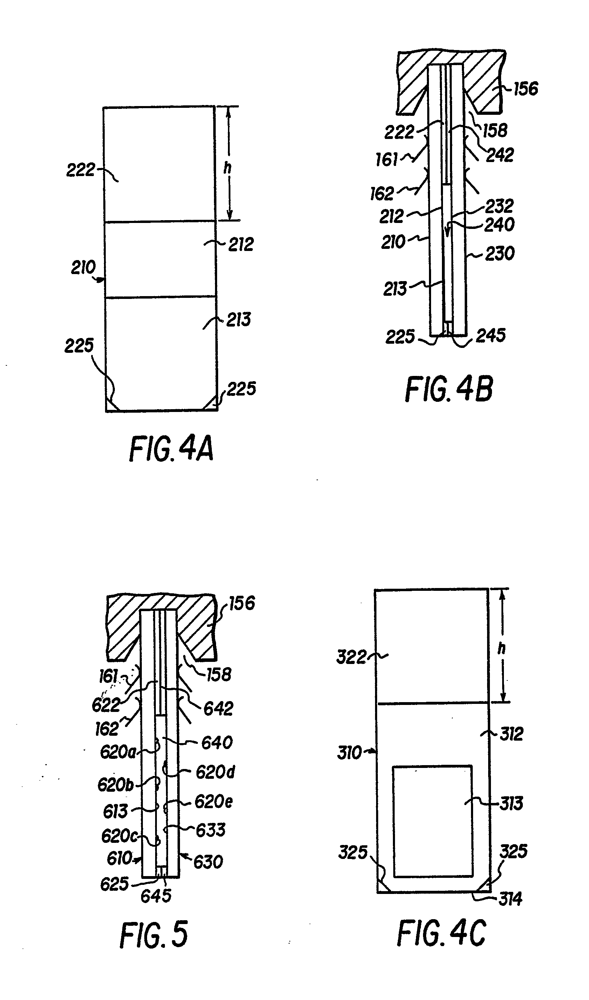

In use, up to thirty slide pairs (with samples on one or both slide of each pair) are inserted into the holder. The holder is then lowered onto a series of liquids, typically liquid reagents. Each liquid may be in the form of a bath or sheet, in the form of individual round droplets supported on a droplet holder (see Figure 7 of U.S. Patent 4,731,335) or in the form of laterally-extending aliquots on a modified droplet holder (see Figures 3A, 3B and 3C of an application of Brigati, USSN 032,875, filed March 31, 1987 now U.S. Patent 4,798,706, issued January 17, 1989). Liquid rises by capillary action into the gap 140 between each first slide 110 and the adjacent second or facing slide 130. See Figure 3A hereof. After the appropriate time of liquid contacting sample on one or both slides, the slide assembly is then lowered on a flat blotter 172, as shown in Figure 3B hereof. The column of liquid 170 is then drawn by capillary action into the blotter 172 so as to evacuate each capillary gap 140. If a droplet holder is used for the particular step, then the process can be individualized so as to treat different slide pairs with different liquids (e.g., different primary antibodies, nucleic acid probes, enzymes or chromogens). After evacuation, the slide assembly can then be contacted by another reagent in the form of droplets, laterally-extending aliquots or a sheet or bath of liquid.

Looking at Figure 3A, the liquid droplet is in hole 166 through elastomeric member 164 on rigid base 162. The lower edges 114 and 134 of slides 110 and 130, respectively, contact the top of the droplet so that the lower edge 142 of gap 140 also contacts the droplet. A rising column of liquid 170 is drawn upward into the gap 140 by capillary action as described more fully in U.S. Patent 4,731,335. It should be understood that, in the present embodiment, liquid in column 170 contacts adsorbent polymeric layers 113 and 133.

Looking at Figure 3B, the lower edges 114 and 134 of slides 110 and 130 contact, at the end of a treating or washing step, a blotter 172 of absorbent material. The liquid is then drawn by capillary action out of gap 140 through its lower edge 142 into blotter 172, where it forms a downwardly and outwardly spreading liquid front 174, causing the column 170 of liquid in gap 140 to fall.

In the course of multiple treatment steps, the pair of slides 110 and 130 are repeatedly contacted with sources of treating liquids (some of which may be discrete droplets as in hole 166) and blotters such as blotter 172. Looking at Figures 2A and 3B, it should be appreciated that coatings 122 and 132, coupled with the rigidity of slides 110 and 130, will usually, but not always, suffice to maintain the thickness of the gap 140 between lower edges 114 and 134 constant. In particular, when lower edges 114 and 134 are pressed down into blotter 172, the blotter 172 will tend to press edges 114 and 134 of slides 110 and 130 together. Such pressure is likely to occur when the resistance of edges 114 and 134 against blotter 172 is relied upon to stop the downward motion of the array of slides as is the case with current models of Fisher Scientific Company's HISTOMATIC slide stainer, CODE-ON version, which employs a solenoid mechanism to stop downward movement when the slide array encounters a resistance.

By providing raised islands 125 and 145 (as seen in Figures 2A, 2B and 2C), edges 114 and 134 are prevented from coming together to close lower edge 142 of gap 140. The function of such raised islands 125 and 145 and alternative configurations of such islands are described more fully in USSN 112,404, the disclosure of which is incorporated herein by reference.

The adsorbent polymeric coatings 113 and 133 do not substantially affect the flow of liquid into and out of gap 140. Thus, comparing present Figures 3A and 3B to Figures 3A and 3B of U.S. Patent 4,731,335, good flow into and out of gap 140 in each case is optimized for a variety of liquids when the gap thickness is within the preferred range of about 150 um to about 250 um. Gaps that are thinner than about 150 um may be difficult to quickly evacuate of certain liquids. Gaps that are thicker than about 250 um may be difficult to fill with certain liquids. Nevertheless, the various range of about 50 to about 500 um, preferably about 100 to about 250 um, and more preferably about 150 to about 250 um and most preferably about 150 um to about 200 um are justified by recognizing that some slide assemblies may be used in applications where either rapid filling or rapid evacuation may not be required, or where only certain classes of liquids are encountered. The more and most preferred ranges of thicknesses impart maximum flexibility to the slide pair.

In this embodiment the gap thickness represents the spacing from one adsorbent polymeric layer 113 to another 133. In certain other embodiments (see Figure 4B) the gap thickness represents the spacing from an adsorbent layer to a glass surface. In certain other embodiments (see Figure 4C), the spacing between two glass surfaces may also be important, as discussed below. In general, adsorbent polymer layers should have a thickness of about 2 to about 50 um, preferably about 5 to about 50 um and more preferably about 10 to about 30 um.

Figure 4A shows the front view of a slide 210 having a top coating 222 and corner spots 225 similar to coating 122 and corner spots 125 of the embodiment of Figure 1. In this second embodiment, adsorbent polymer layer covers only the bottom 31.8 mm of the front glass surface 212 of slide 210. Accordingly, the middle 12.6 mm (76.2 - 31.8 - 31.8) of front planar glass surface 212 is visible in Figure 4A below epoxy coating 222 and above nitrocellulose layer 213.

Figure 4B shows the slide 210 of Figure 4A inserted with a slide 230 into the slot 158 in holder 156 of Figure 2A and of U.S.S.N. 032,874. Clips 161 and 162 force the top coating 242 on glass surface 232 of slide 230 against top coating 222 on slide 210. Below top coating 242, front planar glass surface 232 of slide 230 is uncovered (except for corner spots 245). Accordingly, the bottom 31.8 mm of gap 240 is the thickness from the front of nitrocellulose layer 213 to the glass surface 232. Illustrative thickness are 1 mm (1000 um) for slides 210 and 230, 90 um for top coatings 222 and 242 and 90 um for corner spots 225 and 245 (assuming that the corner spots are not covered by nitrocellulose). Accordingly, glass surface 212 is spaced from glass surface 232 by 180 um.

Nitrocellulose layer 213 can thus be 30 um thick without reducing the thickness of the gap 240 to below the preferred minimum of 150 um. If a continuous nitrocellulose layer is applied from solution (as described below), then it is preferred that its thickness be kept small enough for the gap to be at least 150 um thick (that is a thickness of layer 213 in this embodiment of 30 um or less). Nitrocellulose layer could, however, be a thin filter layer applied directly to surface 212 with an adhesive or otherwise. In the event that a 50 um thick layer was applied in this fashion, the gap thickness would be 130 um, which would still be suitable for many applications. If one wished to restore the gap thickness to 150 um, one need only increase the thickness of epoxy coatings 222, 225, 242 and 245 from 90 um to 100 um.

Figure 4C illustrates a slide 310 according to a third embodiment. Epoxy coating 322 cover the top h portion of the front face 312 of slide 310 (the top 30 mm in this case). Corner spots 325 are again right isoceles triangles of 4 mm maximum height and 4 mm maximum width extending inwardly from opposite corners on front face 312 along lower edge 314 of slide 310. In this case, coatings 322 and 325 have an exemplary thickness of 85 um.

The adsorbent polymeric layer 313 covers a portion of front face 312 of slide 310 which begins 5 mm above lower edge 314. Layer 313 has a width of 20 mm and is centered within the 25.4 mm width of slide 310. Layer 313 extends upward 30 mm so as to terminate 11.2 mm below the bottom edge of epoxy coating 322. An exemplary thickness for layer 313 is 20 um.

If two slides 313 were placed in the slot and clips of Figure 2A, the spacing between front faces 312 would be 190 um (twice the thickness of coatings 322 and 325). The facing nitrocellulose layers would be spaced from each other by a gap thickness of 150 um. Referring to Figure 3A, it should be appreciated that the lower edge of the gap (analogous to 142 in Figure 3A) would in this third embodiment be 190 um thick. That is a satisfactory thickness for liquid to be drawn into the gap. Liquid would rise in a column and contact each layer 313. When the slide assembly was moved to a blotter (such as blotter 172 in Figure 3B), liquid would be drawn out of the gap. Because the gap would be thinnest in the vicinity of layers 313, it is expected that the center of the column might drain more slowly than the edges. Nevertheless, with a minimum gap thickness of 150 um, it is expected that the gap could be rapidly evacuated of various liquids. Especially when the layer 313 has been applied by the preferred method from a solution in an organic solvent (e.g., nitrocellulose applied from a methanol solutions, the amount of liquid that would adhere to layer 313 after evacuation would be expected to be very small. Even when a filter material is used, the liquid that does adhere can be compensated for by increasing the number of various washing steps.

Figure 5 shows two slides 610 and 630 adapted for either a dotting or a blotting assay. In blotting (unlike dotting), the sample is subjected to a geometric separation before transfer to the filter material. Slides 610 and 630 are fitted into the same slide holder. Upper coatings 622 on slide 610 and 642 on slide 630 abut against each other under the compression of clips 161 and 162. Slides 610 and 630 fit at their upper ends into slot 158 in alignment strip 156 of the holder. A thin strip of nitrocellulose material or other membrane filter material 613 covers the inner surface of slide 610 below coating 622. A thin strip of nitrocellulose or other filter material 633 covers the inner surface of slide 630 below coating 642. Slides 610 and 630 need not be glass, and can be a thermoplastic or thermosetting material to which thin strips 613 and 633, respectively, can tightly bind.

The gap between facing surfaces of thin strips 613 and 633 has the thickness of the first distance of U.S. Patent 4,731,335 (generally 50 to 500 micrometers, preferably 150 to 250 micrometers, more preferably 150 to 200 micrometers). If each of thin strips 613 and 633 is 50 micrometers thick, then a gap of 150 micrometers can be created by making each of coatings 622 and 642 a thickness of 125 micrometers. Furthermore, slide 610 can be formed (especially if plastic) as a unitary structure with a top portion extending 125 micrometers further forward than the bottom portion either by molding, by removing material from the lower portion or otherwise. Thin strip 623 can be attached chemically, with adhesive or by heat setting (analogous to ironing on) if slide 610 is of an appropriate material. The entire structure represented by slide 610, coating 622 and layer 623 can also be formed as a laminant, by analogy to Figures 17-20 of U.S. Patent 4,308,02B to Elkins.

In use in a dotting assay, slide 610 is spotted directly with samples (such as cells or cellular extracts of protein or purified DNA) using a manual or automatic pipetting device. The samples are bound to the membrane of filter material on slide 610 by heating, drying, adsorption, absorption or chemical linkage. The thin strips of slides 610 and 630 are then apposed, and their upper ends are placed in slot 158 in alignment strip 156 of the holder to form a capillary action gap similar to that depicted in Figures 3A and 3B.

In use for blotting, slide 610 (and separately slide 630) is blotted against an electrophoretic gel or other separating device so as to transfer previously separated species (e.g., antigens or polynucleotides) onto strip 613 in a defined spatial relationship (e.g., short polynucleotides migrate further into a gel than longer ones). The slide is then removed from the gel or vice versa. The blotted material can then be more firmly attached to the nitrocellulose by drying or heating or by using chemically linking reagents (e.g., glutaraldehyde) if desired. In Figure 5, three specimen spots 620a, 620b and 62c are shown on nitrocellulose strip 613. Two other specimen spots 620d and 620e are shown on nitrocellulose strip 633. These specimen spots, irrespective of whether they were spotted or blotted onto the membrane, can then be exposed to a series of probing, analyzing, washing, treating and developing reagents in a manner analogous to the multi-step process described for tissue specimens in U.S. Patent 4,731,335. At the conclusion of the process, a chromophore would be deposited at the locus of each spot for which the reagents are specific. Thus, for example, a restriction enzyme digest of a DNA source can be electrophoresed, blotted and probed to see what lengths of restriction fragments have the sequence probed for. Attachment of binding pair members can be performed in advance by conventional linking chemistry (e.g.carbodiimide chemistry). Alternatively, loci on the membrane can be spotted to have defined pre-bound chemically reactive groups (e.g., N-hydroxysuccinimide, azido, or aldehyde groups) which directly bind to reactive moieties of sample materials (e.g., amino, carboxy, sulfydryl, purine, pyrimidine or hydroxyl groups) when the sample comes in contact with the loci in the course of filling the capillary action gap with sample. In these cases (unlike those described previously), the slide assembly of slides 610 and 630 is formed without a sample pre-bound on either slide. Either as the initial step or after one or more preparative washing steps (by capillary drawing and evacuation as in Figures 3A and 3B), a sample material such as a drop of serum is drawn up into the gap between strips 613 and 633 by capillary action (in a fashion analogous to that shown in Figure 3A). The assembly is then incubated at an appropriate temperature or temperature profile. If binding occurs, then analyte material in the sample would bind at the appropriate loci of strip 613 or 633. For example, if a viral antigen is bound at one locus on nitrocellulose strip 613, any antibody to that antigen in the serum sample would bind at that locus. Other antibodies in the serum sample would not. The gap could then be evacuated and subjected to various washing steps corresponding to steps 18A-19B of the staining procedure at columns 13-15 of U.S. Patent 4,731,335. Next, a labeled antibody could be brought in which is specific for any human antibody (e.g., rabbit anti-human Ig) or is specific for certain human antibodies (e.g, goat anti-human IgG). The labeled antibody can be directly readable (e.g., a fluorescent tag), or serve as a site for further attachment of readable label (e.g., biotin serving as an affinity site for avidin-enzyme, avidin-fluor or ABC reagents) or an enzyme (e.g., horseradish peroxidase or alkaline phosphatase). If enzyme is then present where serum antibody had originally bound, then (after further washing) chromogen can be brought in (by analogy to steps 23A to 25B of such staining procedure). At the conclusion of such process, slide 610 can be examined by the naked eye, by a microscope or by an electronic visualization device to determine if color is present at that spot as an indication if (and semiquantitatively how much of) the looked for antibody was present in the serum sample.

The advantage of this strategy is that the loci can be compared to the surrounding membrane as a control, or to other spots to provide a simple reference system for quantitation. For example, such other spots could have pre-bound known amounts of analyte, in either a single or multiple amounts. Alternatively, another spot could have pre-bound negative controls, or a pre-bound binding partner for a binding pair member with which the sample is spiked to serve as a calibrator or internal control for the read-out.

It should be appreciated that a single sample can be simultaneously assayed for antibodies against different antigens, if these antigens are pre-bound as spots in a known pattern on strips 613 and 633. Alternatively, identical spots can be formed on strips 613 and 633 to be exposed to the same serum sample and washed. The slides can then be removed from the holder and rearranged in different slide pairs so that, for example, slide 610 is exposed to anti-human Ig antibody and then developing reagents while slide 630 is exposed to anti-human IgG antibody (or anti-human IgM antibody) and then developing reagents. In a third format, a single sample can be analyzed for many antigens if antibodies to these antigens are pre-bound as spots in a known pattern on strips 613 and 633. In a fourth format, specific nucleic acid sequences are spotted at defined loci on the strips 613 and 633 for hybridization assays with prepared samples, such as those wherein sample nucleic acid has been exposed, labeled and placed in suitable hybridization media for denaturation and hybridization. Such fourth format can also be used for nucleic acid sandwich assays.

In such methods of use, various steps (incubation, enzyme reaction) can be accelerated with heat (as described in U.S. Patent 4,731,335) or with radiation. With appropriate holders (not made of metal) microwave radiation can be used. Methods employing infrared radiation (which can be used even if the holder is metal) are described in USSN 168,173 of Koebler, Cuomo and Brigati, filed March 15, 1988, copending and commonly-assigned.

The method of the present invention represents a generalized method for applying a thin layer of an adsorbent material such as nitrocellulose to a flat glass surface. It has particular application in forming the thin adsorbent polymer layers of preferred embodiments of the device and slide assembly of the present invention (such as layers 113, 133, 213, 313, 613 and 633 of the various Figures).

The present method begins with a positively charged glass surface. If the adsorbent polymer to be applied bears a net negative charge (as does nitrocellulose), then no further pretreatment of the glass surface beyond imparting the positive charge is required. As discussed below in connection with polyacrylamide as the polymer, it may be necessary in other cases to further derivatize the glass surface, as with glutaraldehyde, neutral buffered formalin, or other stable linking agent.

Positively charged glass surfaces may be formed by known methods, such as those developed by Howard Weetall of Corning Glass Works. For example, aminoalkylsilane compounds may be applied to glass surfaces in solvents such as acetone. The application of one such compound (aminopropyltriethoxysilane or APTS) as an aqueous solution is described in an example, below.

The adsorbent polymer should be applied as a solution (without undissolved polymer suspended) to the positively charged glass surface. Polar organic solvents should be used. For nitrocellulose, methanol is the preferred solvent. It causes the layer to be continuous and transparent over the thickness range of 2 to 50 um. Solvents such as isopropanol and ethanol have been found thus far to be less preferred, because the layer is not as transparent and may be somewhat uneven. In limited experiments, acetone has been generally unsatifactory, because discontinuous layers of nitrocellulose were formed in these limited experiments. It is possible, however, that in these experiments, not all of the nitrocellulose was dissolved in the acetone, or the drying of the acetone was not performed in an optimal fashion. Thus, insufficient information is available to completely exclude any polar organic solvent, even acetone.

Dilute solutions are generally used. For nitrocellulose in methanol, concentrations of 1% and 2% by weight have been found effective, and somewhat higher and lower concentrations would also be suitable. The thickness of the layer one forms can be controlled by adjusting the concentration of the solution and, in some geometries, also the amount of solution applied. If the glass is dipped in the solution, then concentration would be the primary basis for control, with more concentrated solutions leading to a thicker layer.

The drying of the solution on the glass layer can be expedited by a variety of heat or radiation sources. Microwaves were used in some of the experiments performed to date. The glass surface is normally held horizontal during drying to enable the layer to form evenly.

For polyacrylamide, one might use a 1% solution of an electrophoresis grade of polyacrylamide in 0.1 M phosphate buffer of pH 7.0 - 7.5. The positively charged glass surface would be treated with a 1% solution of glutaraldehyde in 0.1 M phosphate buffer of pH 7.0 - 7.5 and then rinsed and dried before applying the polyacrylamide solution. After the polyacrylamide solution had dried, the slide might be rinsed with any aqueous solution.

## EXAMPLE S

PROBE-ON slides having the general configuration of Figures 5A and 5B of U.S.S.N. 033,073 (the embodiment of present Figure 1 without the layer 113) were rinsed with distilled water and then dipped in a 2% solution of APTS (Cat. No. A3648 from Sigma Chemical) in distilled water for two minutes. The slides were then rinsed by dipping in five successive one liter vessels of distilled water and dried in an 80 degree C convection oven and then at room temperature in air.

The slides with positively charged surfaces made by such a treatment were then dipped into conical 50 mL centrifuge tubes containing a solution of nitrocellulose in methanol. That solution had been made by cutting up two nitrocellulose membranes (88 mm by 88 mm Schleicher and Schuell BA-85) of a total weight of about 1.1 g and dissolving the pieces in a total of 30 ml of absolute methanol by rotary stirring. The solution could be filtered, if desired, to remove any undissolved nitrocellulose before use.

The slides removed from the centrifuge tubes were then dried in a horizontal orientation in a microwave oven for thirty seconds. Microscopic examination of the dried slides showed an optically clear layer of approximately 5 - 10 um thickness uniformly coating all of the glass and epoxy surfaces that had been immersed in the methanol solution. When this process was repeated using methanol solutions to which a food dye had been added, the complete coverage and evenness of the layer was confirmed. Thickness of the coating could also be adjusted by applying additional layers of nitrocellulose/methanol solution to the first application.

Slides, now having the characteristics described above in connection with Figures 1, 2A, 2B and 2C, but with nitrocellulose layers 113, 133, 125a and 145a being approximately 10 um, were then evaluated in applications as follows.

The binding capacity of the nitrocellulose layered glass was initially tested using (A) biotin labelled goat anti-rabbit IgG, (B) horse radish peroxidase, and (C) alkaline phosphatase. The biotin labeled goat anti-rabbit IgG was diluted in 0.01 M NaCl in distilled water pH adjusted to 7.5 with dilute NaOH. The horseradish peroxidase was diluted with distilled water. The alkaline phosphatase was diluted in 0.01 M TrisHCl buffer containing 0.01 M zinc chloride and 0.01 M magnesium chloride (adjusted to pH 8.5 with dilute NaOH). The reagents were spotted in 5 ul drops onto the nitrocellulose layer of the slides and allowed to air dry. All three solutions were begun at 10 mg/ml and diluted in serial 10 fold dilutions in the correct diluent. Five 5 ul spots containing 50 ug, 5 ug, 0.5 ug, 0.05 ug and 0.005 ug were therefore spotted in a row on a single slide.

For the two enzymes (Cases (B) and (C)), only the appropriate chromagen was applied in the gap to the dried spots, except for washing steps. For horseradish peroxidase (Case (B)), a solution of 0.5 mg/ml of diaminobenzidine in 0.01% hydrogen peroxide diluted in 1X Automation Buffer (described below) was used. Development occurred in five minutes at room temperature. The spots containing 5 ug and 0.5 ug enzyme were visualized in a light microscope.

For alkaline phosphatase (Case (C)), a BCIP/NBT solution was used. The spots containing 5 ug, 0.5 ug and 0.05 ug enzymes were seen in the light microscope after two hours of development at 37 degrees C.

The biotin labeled goat anti-rabbit dilution dots (Case (A)) were visualized in a series of steps. First, the slides were placed in a capillary gap formation and prewet with 1X Automation Buffer. This buffer (from Biomeda Corporation) is a Tris based buffer system containing detergent to increase capillary flow. The slides were then treated for 20 minutes at room temperature with a 1:200 dilution of a 1 mg/ml solution of peroxidase conjugated avidin (BIOSTAIN from Biomeda Corporation) diluted in 1 X Automation Buffer containing 1% Bovine Serum Albumin (Sigma Fraction V). The slides were washed three times in 1X Automation Buffer and detected with a 0.5 mg/ml solution of DAB dissolved in 1X Automation Buffer containing 0.1% hydrogen peroxide for 5 minutes at 37 degrees C. The slides were then washed in 1X Automation Buffer and allowed to air dry. Three of the five dots of biotin labeled goat anti-rabbit antibody could now be seen.

## Antigen Localization

To test antigen localization, purified Beta subunit of human HCG was diluted to 100 units/ml in distilled water. Three 5 ul spots of ten-fold dilution were applied to the nitrocellulose layer of a slide. The dots therefore contained 0.5, 0.05 and 0.005 units of Beta subunit HCG. The slides containing the dots were, in sequence: (A) placed in capillary gap formation, (B) washed three times in 1X Automation Buffer, (C) incubated with a 1:200 dilution of rabbit anti-Beta subunit of HCG (20 minutes at 37 degrees C), (D) washed twice in 1X Automation Buffer, (E) incubated with a 1:200 dilution of horseradish peroxidase conjugated goat anti-rabbit antibody (20 minutes at 37 degrees C, (F) washed twice in 1X Automation Buffer, (G) treated with a 0.5 mg/ml DAB solution in 1X Automation Buffer containing 0.1% hydrogen peroxide for 5 minutes at 37 degrees C, (H) washed twice in 1X Automation Buffer, and (I) removed from the slide holder. The slides were then air dried and examined with a standard light microscope. The 0.5 unit spot of beta-HCG could be visualized under the microscope.

## DNA Immobilization

To test DNA immobilization, 10 ug, 1 ug and 0.1 ug of human placental DNA (Sigma) biotinylated by nick translation before use were immobilized onto nitrocellulose layers of a slide prepared as above. The biotinylated DNA was spotted in distilled water with 0.1 M phosphate buffer and pH 7.5 and allowed to dry by baking 4 hours at 80 degrees C (see Leary, Brigati and Ward, Proc. National Acad. Sci. vol. 80, pp 4045-4049 (1983) and Ward et al, U.S. Patent 4,687,732). The nitrocellulose coated slides were then placed in capillary action formation and then, in sequence: (A) wet twice with 1X Automation Buffer, (B) treated with a 1:200 dilution of alkaline phosphate conjugated avidin (DAKO Corporation) in 1X Automation Buffer containing 1% BSA, 0.1 mg/ml zinc chloride and 0.1 mg/ml magnesium chloride (20 minutes at 37 degrees, (C) washed twice in 1X Automation Buffer, (D) detected by incubating with a BCIP/NBT solution for 2 hours at 37 degrees C, (E) washed twice in 1X Automation Buffer, and (F) removed from the holder. The slides were then air dried and examined under a light microscope. The 10 ug and 1 ug spots of biotinylated DNA were visualized as blue dots. The 10 ug dot was stronger than the 1 ug dot.

The binding of the slides for cells was then evaluated for the untreated glass, the slides after treatment with APTS, and the slides after treatment with APTS and nitrocellulose/methanol as described above. The APTS treated glass was used either as is, or after further treatment with 1% glutaraldehyde dissolved in 0.1M phosphate buffer, pH 7.5, or 1% neutral buffered formalin.

Cells of various types were centrifuged at 2000 rev/min for 5 minutes, applied to the slides and either air dried onto the slides or fixed in 50% ethanol for one minute and then air dried. The best adherence of various cell types was with aldehyde treated APTS glass, then the nitrocellulose treated APTS glass, then the APTS glass and finally the untreated glass. The nitrocellulose treated APTS glass maintained the adherence of cells well after washing in 1X Automation Buffer. By contrast, the APTS glass adhered some cell suspensions better than others.

**Claims**

1. A device comprising:

a) a transparent sheetlike object having a planar front face, a linear lower edge and a thickness of about 0.5 to about 5.0 mm,

b) a rigid polymer coating on a first portion of the planar front face of a thickness about 52 um to about 500 um, and

c) an adsorbent polymer layer on a second portion of the planar front face of a thickness about 2 to about 50 um, the adsorbent polymeric layer having been deposited as a solution of a cellulosic or acrylamide polymer in a polar organic solvent, and the thickness of the rigid polymer coating being at least 50 um greater than the thickness of the adsorbent polymer layer.

2. The device of claim 1 wherein the rigid polymer coating has a thickness of about 75 um to about 125 um.

3. The device of claim 1 or 2 wherein the adsorbent polymer layer has a thickness of about 5 to about 50 um.

4. The device of any prior claim wherein the adsorbent polymer layer is nitrocellulose.

5. The device of any prior claim wherein the first portion of the planar front face includes a portion distal from the linear lower edge and the second portion of the planar front face includes a portion adjacent to the linear lower edge.

6. The device of any prior claim wherein the transparent sheetlike object is a microscope slide and the adsorbent polymeric layer is an optically transparent nitrocellulose layer.

7. A slide assembly comprising:

a) a first rigid slide having a planar front face, a linear lower edge and a thickness about 0.5 to about 5.0 mm,

b) a second rigid slide having a planar front face, a linear lower edge and a thickness about 0.5 to about 5.0 mm,

c) a first adsorbent polymer layer on a portion of the planar front face of the first rigid slide, the adsorbent polymeric layer having been deposited as a solution of a cellulosic or acrylamide polymer in a polar organic solvent, and

d) spacer means for maintaining the first and second slides in a parallel and substantially vertical alignment, with the first adsorbent polymer layer being spaced from the second rigid slide by a gap of about 50 to about 500 um in thickness.

8. The slide assembly of claim 7 wherein the spacer means comprises abutting rigid polymer coatings on the planar front faces of the first and second rigid slides of a total thickness of about 52 to about 500 um.

9. The slide assembly of claim 7 or 8 wherein the first adsorbent polymer layer has a thickness of about 2 to about 50 um and the portion of the planar front face of the second rigid slide which is adjacent to the first adsorbent polymer layer is uncoated and is spaced about 50 to about 450 um from the first adsorbent polymer layer.

10. The slide assembly of claim 9 wherein the uncoated portion of the planar front face of the second rigid slide is spaced from the first adsorbent polymer layer by a gap of about 150 to about 250 um.

11. The slide assembly of claim 7 or 8 wherein a second adsorbent polymeric layer covers a portion of the planar front face of the second rigid slide and wherein the first and second adsorbent polymeric layers each have a thickness of about 2 to about 50 um.

12. The slide assembly of claim 11 wherein the first adsorbent polymeric layer is spaced from the second adsorbent polymeric layer by a gap of about 150 to about 250 um.

13. A method for applying a very thin layer of an adsorbent polymeric coating on a glass surface which comprises the steps:

a) applying a solution of an adsorbent cellulosic or acrylamide polymer in a polar organic solvent to a positively charged glass surface, and

b) drying the solution of step (a) on the positively charged glass surface, and

the concentration of the adsorbent polymer in the solution and the amount of solution applied being such that the dried layer formed by the drying step (b) is about 2 to about 50 um in thickness and forms a continuous coating.

14. The method of claim 13 wherein the adsorbent polymer is nitrocellulose and the polar organic solvent is methanol.

15. The method of claim 13 or 14 wherein the solution is applied to a first portion of a planar glass surface and wherein a second portion of the planar glass surface is covered by a rigid polymer coating of about 52 to about 250 um thickness.

16. The method of claim 13 or 14 or 15 wherein the dried layer formed by the drying step (b) is about 5 to about 50 um in thickness.

FIG.1

FIG.2A

FIG.3A

FIG.3B

FIG.2B

FIG.2C

FIG. 4A

FIG. 4B

FIG. 5

FIG. 4C